# EUROPEAN PATENT APPLICATION

(11) **EP 4 327 723 A1**
(43) Date of publication of application: **28.02.2024**
(21) Application number: 22192419.4
(22) Date of filing: 26.08.2022
(51) Int. Cl.: A61B 3/12, A61B 3/00, A61B 3/14

(54) **RETINA TISSUE IMAGE ACQUISITION**

(71) Applicant: EarlySight SA, 1202 Genève (CH)
(72) Inventor: Laforest, Timothé, 1202 Genève (CH); Kunzi, Mathieu, 1202 Genève (CH)
(74) Representative: KATZAROV S.A.

(57) **Abstract**

The present relates to a method for imaging the retina tissue of a patient comprising:
- i) providing a set of illumination sources comprising at least a first illumination source configured for emitting oblique illumination through the scattering tissue of the eye toward the retina tissue, for instance transscleral or transpalpebral illumination, and at least a second illumination source configured for emitting transpupillary illumination toward the retina tissue;
- ii) defining a predetermined illumination sequence for controlling the oblique illumination and the transpupillary illumination of the retinal tissue by selectively activating said first illumination sources and said second illumination source from said set of illumination sources;
- iii) illuminating the retinal tissue according to the predetermined illumination sequence;
- iv) synchronising the sensor device according to the predetermined illumination sequence to ensure the acquisition of at least one image of the retina during illumination by the activated illumination sources.

## Description

### Technical Field

The present invention relates to a method for imaging the retina tissue of a patient. The invention further relates to a system for retinal tissue imaging.

### Background of the art

Various approaches have been proposed, to image the different types of cells and structures in the retina with microscopic resolution.

Optical coherence tomography has the ability to image simultaneously several layers of the retinal tissue, but its lateral resolution prevents the observation of single cells. When coupled to adaptive optics system, it has shown to reach acceptable resolution to see single cells, but the limitations are a small field of view and long acquisition time, making it impossible to be used in patients.

Therefore, there is a need to provide an improved method for imaging different types of cells from the retinal tissue in a quick and efficient manner.

### Summary of the invention

The above problems are solved or at least limited by the system and the method according to present invention.

The invention concerns a method for imaging the retina tissue of a patient, the method comprising:
- i) providing a set of illumination sources comprising at least a first illumination source configured for emitting oblique illumination through the scattering tissue of the eye toward the retina tissue, for instance transscleral or transpalpebral illumination, and at least a second illumination source configured for emitting transpupillary illumination toward the retina tissue;
- ii) defining a predetermined illumination sequence for controlling the oblique illumination and the transpupillary illumination of the retinal tissue by selectively activating at least said first illumination sources and said second illumination source from said set of illumination sources;
- iii) illuminating the retinal tissue according to the predetermined illumination sequence;
- iv) providing a sensor device and synchronising the sensor device according to the predetermined illumination sequence to ensure the acquisition of at least one image of the retina during illumination by the activated illumination sources.

In another aspect, the invention concerns a system for imaging the retina tissue of a patient, the system comprising an illumination device and a sensor device, the illumination device being configured for illuminating the retina tissue while the sensor device acquires images of the illuminated retina tissue,
the system being characterized in that the illumination device comprises
a set of illumination sources comprising at least a first illumination source configured for emitting oblique illumination through the scattering tissue of the eye toward the retina tissue, for instance transscleral or transpalpebral illumination, and at least a second illumination source configured for emitting transpupillary illumination toward the retina tissue,
the system further comprising a control module configured for controlling the oblique illumination and the transpupillary illumination of the retinal tissue by selectively activating at least said first illumination sources and said second illumination source from said set of illumination sources according to a predetermined illumination sequence,
said control module being further configured for synchronising the sensor device according to the predetermined illumination sequence to ensure the acquisition of at least one image of the retina during illumination by said activated illumination sources.

### Description of the invention

In a first aspect, the invention concerns a method for imaging the retina tissue of a patient, the method comprising:
- i) providing a set of illumination sources comprising at least a first illumination source configured for emitting oblique illumination through the scattering tissue of the eye toward the retina tissue, for instance transscleral or transpalpebral illumination, and at least a second illumination source configured for emitting transpupillary illumination toward the retina tissue;
- ii) defining a predetermined illumination sequence for controlling the oblique illumination and the transpupillary illumination of the retinal tissue by selectively activating at least said first illumination sources and said second illumination source from said set of illumination sources;

- iii) illuminating the retinal tissue according to the predetermined illumination sequence;
- iv) providing a sensor device and synchronising the sensor device according to the predetermined illumination sequence to ensure the acquisition of at least one image of the retina during illumination by the activated illumination sources.

The advantage of combining an oblique illumination and a transpupillary illumination of the same tissue is to selectively provide contrast to the structures of interest. Indeed, the retina structures are sensitive to the light orientation and they reflect, transmit, absorb, or scatter light differently depending on the illumination orientation. For instance, photoreceptors strongly reflect transpupillary illumination but transmit oblique illumination, so that the oblique illumination reaches the underlying layers such as retinal pigment epithelium. Consequently, imaging of photoreceptors is optimally performed with transpupillary illumination while retinal pigment epithelium is imaged with oblique illumination.

Combining an oblique illumination and a transpupillary illumination in a single acquisition sequence enables the imaging of various structures in a small time frame and permits their observation on the same zone and same time or almost same time within the patient's eye. It creates a snapshot of various structures, each of them visible in a separate image. The images can be later analysed and compared efficiently because they cover the same zone and were taken at the same time. Computing ratio of the number of a first structure versus the number of a second one per surface area is facilitated with the present invention.

The predetermined pattern allows to select the illumination source to control which illumination source is selectively activated to illuminate the retina, for instance the first illumination source and the second illumination source successively, or simultaneously.

The sensor device (i.e. detector, for instance a camera) is synchronised according to the predetermined illumination sequence to ensure the acquisition of at least one image of the retina during illumination by the activated illumination sources. The point is to acquire at least one image of the retina during illumination by at least one illumination source. For instance:
- at least one image for each activated illumination sources; for instance at least one image when the retina is illuminated by the oblique illumination source and at least another image when the retina is illuminated by the transpupillary illumination source;
- at least one image when the retina is illuminated by all the activated illumination sources simultaneously, for instance at least one image when the retina is illuminated by the oblique illumination source and by the transpupillary illumination source

Preferably, the illumination sequence comprises pulses of illumination. Preferably, all the pulses of an illumination sequence have the same duration. Alternatively, at least some of the pulses of an illumination sequence have different duration. Preferably, a pulse has a duration comprised between about 1 ms to 100 ms, more preferably between about 1 ms and 75 ms, more preferably between about 1 ms and about 50 ms, more preferably between about 1 ms and about 25 ms, more preferably between about 1 ms and about 10 ms.

Preferably, the total duration of the predetermined illumination sequence is comprised between about 1 sec to about 10 min, more preferably between about 1 sec to about 5 min, more preferably between about 1 sec to about 1 min, more preferably between about 1 sec to about 45 sec, more preferably between about 1 sec to about 30 sec, more preferably between about 1 sec to about 10 sec, more preferably between about 1 sec to about 5 sec, more preferably between about 2 sec to about 4 sec, more preferably between about 2 sec to about 3 sec.

Preferably, the sensor device comprises one detector (single detector). This allows using the illumination sequence to create multiple images of multiple retinal structures on the same detector.

Preferably the sensor device comprises several detectors, preferably each activated illumination source is synchronized with its own detectors. For instance, a first detector is synchronized with the first illumination source whereas a second detector distinct from the first detector is synchronized with the second illumination source This allows creating multiples images of retinal structures on different detectors.

In a preferred embodiment, the method further comprises:
- setting a focusing plane of the sensor device during image acquisition at a specific depth in the retinal tissue, the focal plane being chosen depending on the type of structure of the retinal tissue to be imaged;
- defining a predetermined illumination sequence for each focusing plane
Interestingly, when the depth of imaging is at a focusing plane comprising the RPE and photoreceptors, the oblique illumination provides an image of the RPE cells, whereas the transpupillary illumination provides an image of the photoreceptors. Indeed, because these two layers are on top of each other and within the depth of field (i.e. focusing plane) of the imaging system, they can be seen on a camera sensor with a single focusing plane just by selecting the correct illumination sources. This is possible because of the relatively large depth of field of a full-field (flood illuminated) camera imaging method/system.

For instance :
- mode 1) one focusing plane and a predetermined pattern where at least two illumination sources are activated and illuminate the retina alternatively to image two distinct types of structure located in the same plane or within the depth of field;
- mode 2) at least two focusing planes and a predetermined pattern where at least two illumination sources are activated and illuminate the retina alternatively and in synchronization with the change of focusing planes. For instance, a first illumination being used to image structures present at a first focusing plane and a second illumination being used to image structures present at a second focusing plane;
- mode 3) at least two focusing planes and a predetermined pattern where at least two illumination sources are activated and illuminate the retina simultaneously, each illumination sources being synchronized with a detector (i.e. sensor device) adjusted to the corresponding focusing plane. For instance, a first illumination being used to image structures present at a first focusing plane and a second illumination being used to image structures present at a second focusing plane;

Preferably, the structure is chosen among the list comprising the retinal pigment epithelium cells, the photoreceptors, the retinal nerve fibre layer, the retinal vessels, the retinal capillaries, the ganglion cells, the microglia, the amacrine cells, the lamina cribrosa, the optic disc, the choriocapillaris, the choroidal vessels, the choroid and the inner retina.

Preferably, the method further comprising :
- Acquiring at least two images per activated illumination source;
- Processing the acquired images to provide at least one finale image provided by the first illumination source and at least one finale image provided by the second illumination source.

Preferably, the predetermined illumination sequence comprises an alternate activation of at least two illumination sources from the set of illumination sources, preferably of at least the first illumination source and the second illumination source. Alternating between the two sources allows to selectively illuminate the retinal tissue with a preferred orientation and take benefit of the anisotropy of certain retina structures to enhance the contrast of some structures depending on the orientation of the illumination. Synchronizing the imaging sensor (i.e. detector) with the source activation enables to have a first kind of structures visible on a first image thanks to the first source orientation and a second kind of structure visible on a second image thanks to the second source orientation.

Preferably, the predetermined illumination sequence comprises a simultaneous activation of at least two illumination sources from the set of illumination sources, preferably of at least the first illumination source and the second illumination source. Combining simultaneously at least two sources having different orientations creates a broader and more uniform illumination of the retina tissue allowing to image with better contrast some types of structures that are not or little sensitive to the illumination orientation. It increases the imaging signal and reduce the imaging noise.

In one embodiment, the predetermined sequence comprises at least 1 ms between each acquired image.

Preferably, at least two illumination sources from the set of illumination sources have at least one identical optical parameter chosen among wavelength, spectrum, power on the retina.

Preferably, at least two illumination sources from the set of illumination sources have different wavelengths. Preferably, at least two illumination sources from the set of illumination sources have the same wavelength.

Preferably, at least two illumination sources from the set of illumination sources emit in the infrared spectrum, preferably between about 700 nm and about 1000 nm.

Preferably, the images are acquired at a constant focal plane.

The invention also relates to a system for imaging the retina tissue of a patient, the system comprising an illumination device and a sensor device, the illumination device being configured for illuminating the retina tissue while the sensor device acquires images of the illuminated retina tissue,
the system being characterized in that the illumination device comprises
a set of illumination sources comprising at least a first illumination source configured for emitting oblique illumination through the scattering tissue of the eye toward the retina tissue, for instance transscleral or transpalpebral illumination, and at least a second illumination source configured for emitting transpupillary illumination toward the retina tissue,
the system further comprising a control module configured for controlling the oblique illumination and the transpupillary illumination of the retinal tissue by selectively activating at least said first illumination sources and said second illumination source from said set of illumination sources according to a predetermined illumination sequence,
said control module being further configured for synchronising the sensor device according to the predetermined illumination sequence to ensure the acquisition of at least one image of the retina during illumination by said activated illumination sources.

The particular advantages of the system are similar to the ones of the method of the invention and will thus not be repeated here.

Preferably, the system further comprises a correction module for correcting the optical aberrations of the eye of the patient, for instance chosen among a wavefront sensor, a wavefront corrector.

Preferably the system further comprises a processing module for processing the acquired images from the sensor device, the processing module being configured for processing images acquired from each illumination source to provide at least one finale image for each illumination source.

Preferably the system comprises a single detector and the illumination sequence comprises an alternate activation of at least two illumination sources from the set of illumination sources.

Preferably, the system comprises one detector per illumination source and the illumination sequence comprises an alternate activation of at least two illumination sources from the set of illumination sources.

As used herein, the word "means" (singular or plural) preceded or followed by a function can be replaced by the word "unit" or " module". For instance "computation means" can be replaced by "computation module" or "computation unit".

The embodiments describe for the method also apply to the system according to the present invention mutatis mutandis.

### Brief description of the drawings

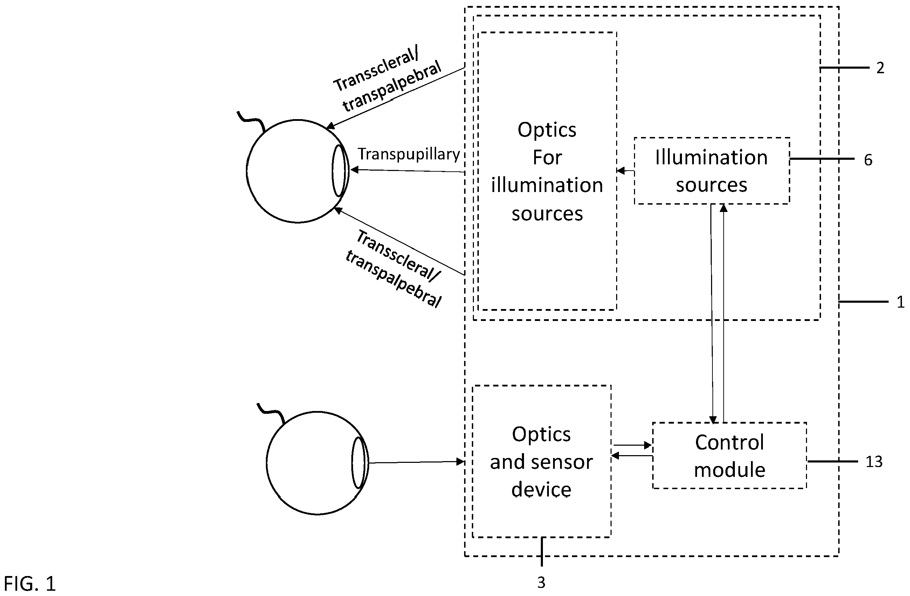

Further particular advantages and features of the invention will become more apparent from the following non-limitative description of at least one embodiment of the invention which will refer to the accompanying drawings, wherein
- Figure 1 illustrates a transscleral illumination of the retinal tissue;
- Figure 2 illustrates a transpalpebral illumination of the retinal tissue;
- Figure 3 illustrates three illumination sources;
- Figure 4 illustrates various illumination sources;
- Figures 5 to 8 illustrates various predetermined illumination sequences;

### Detailed description of the invention

The present detailed description is intended to illustrate the invention in a non-limitative manner since any feature of an embodiment may be combined with any other feature of a different embodiment in an advantageous manner. The present invention is not limited to the embodiments described below.

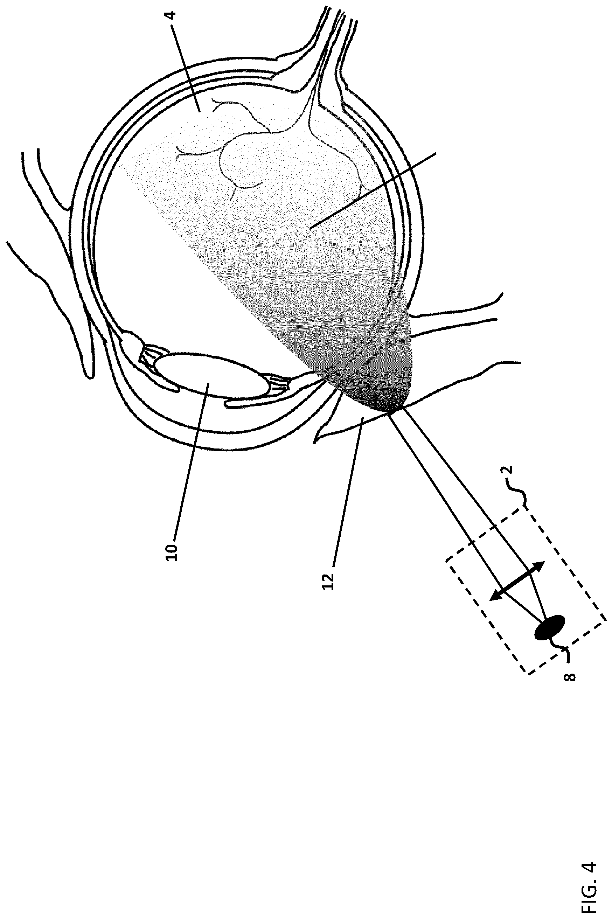

Figure 4 represents an embodiment of a system 1 imaging the retina tissue of a patient according to the present invention but the invention is not limited to this example. The system 1 comprises an illumination device 2 and a sensor device 3, the illumination device 2 being configured for illuminating the retina tissue 4 while the sensor device 3 acquires images of the illuminated retina tissue 4 of the eye 5.

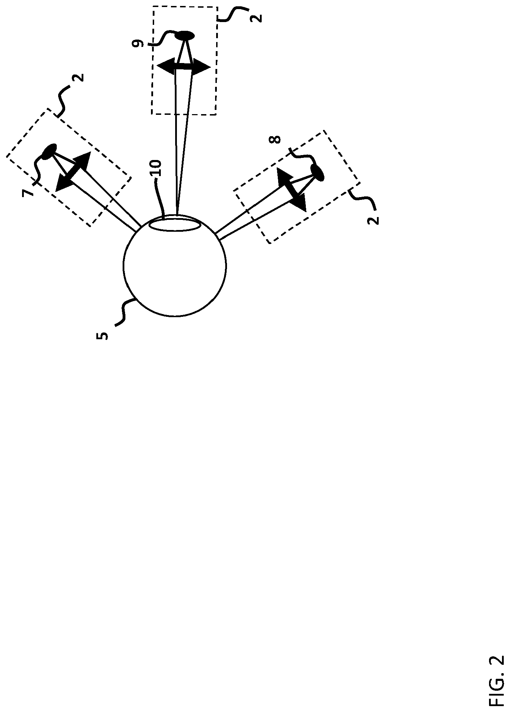

The illumination device 2 comprises a set of illumination sources 6. In this example, the set of illumination sources 6 can comprise illumination sources 6 chosen among a transscleral illumination source 7, a transpalpebral illumination source 8, a transpupillary illumination 9, all illumination sources 6 emitting toward the retina tissue 4 of the eye 5 as shown in figure 2.

For transpupillary illumination, a transpupillary illumination source 9 emits light toward the pupil 10 of the eye 5 to reach the retina tissue 4.

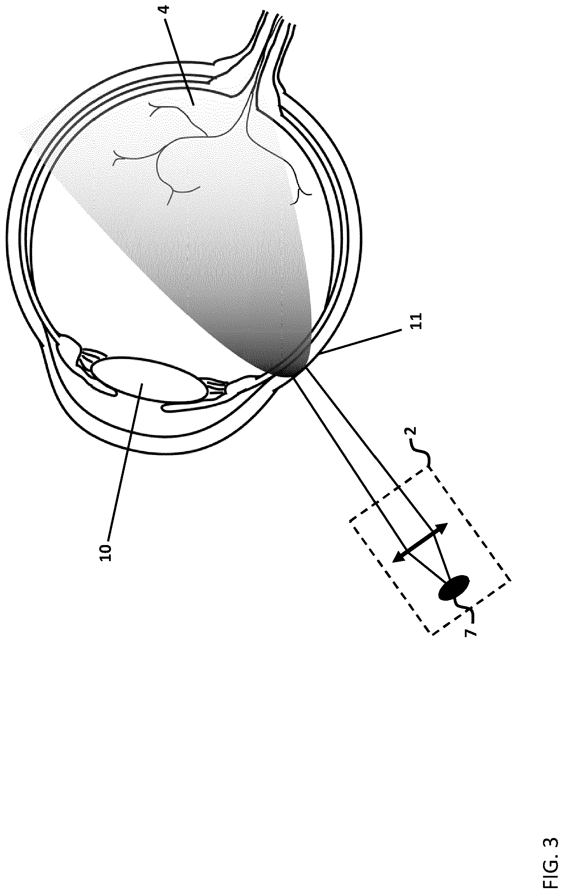

For transscleral illumination, the transscleral illumination source 7 emits light toward the sclera 11 of the eye 5 to reach the retina tissue 4 as illustrated in figure 3.

For transpalpebral illumination, the transpalpebral illumination source 8 emits light toward the surrounding skin 12 of the eye 5 to reach the retina tissue 4 as illustrated in figure 4.

The illumination device 2 further comprises a control module 13 configured for controlling the illumination sources 6, namely the transscleral illumination source 7, the transpalpebral illumination source 8 and the transpupillary illumination source 9 by selectively activating the illumination sources 6 from the set of illumination sources according to a predetermined illumination sequence. The control module is further configured for synchronising the sensor device 3 according to the predetermined illumination sequence to ensure the acquisition of at least one image of the retina 4 during illumination by said activated illumination sources 3.

The illumination sources produce light that is projected on the eye thanks to illumination optics. Before reaching the detector, the light going out of the eye pupil (as a result of the illumination) is collected by optics that create images of the retina on the detector.

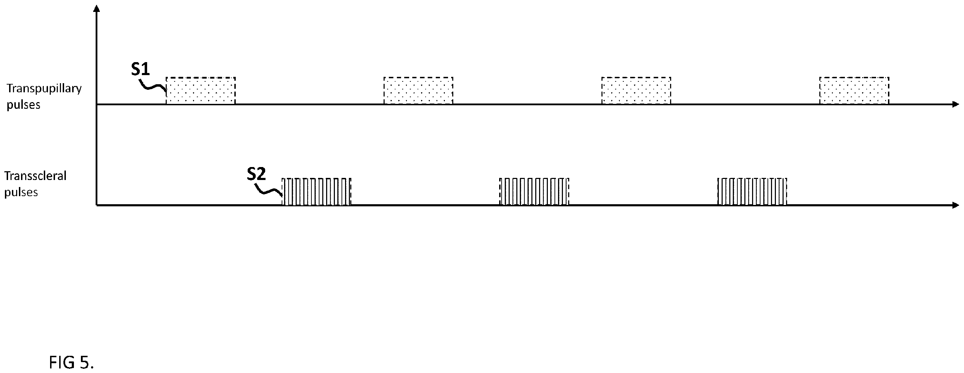

Figures 5 to 8 illustrate various embodiments of predetermined illumination sequences but the invention is not limited to the illustrated embodiments.

Figure 5 illustrates a first embodiment of a predetermined illumination sequence where the illumination is provided by an illumination device 2 comprising a transscleral illumination source S2 and a transpupillary illumination source S1 illuminating the retina tissue 4. The images are acquired by a single detector (ie. sensor device 3). The predetermined illumination sequence comprises transpupillary pulses and transscleral pulses in alternance. This allows for instance the imaging of photoreceptors (with S1) and retinal pigment epithelium (with S2) on the same retina zone and same focusing plane and on two distinct images.

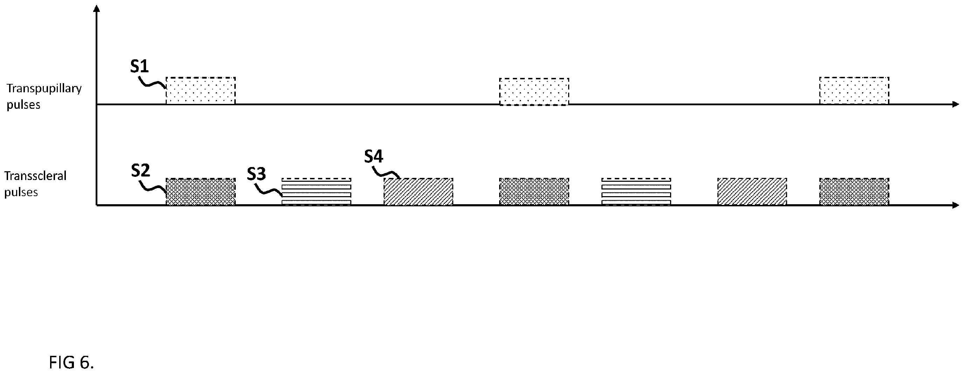

Figure 6 illustrates a second embodiment of a predetermined illumination sequence where illumination is provided by an illumination device 2 comprising a transpupillary illumination source S1 and three transscleral illumination sources S2, S3 and S4 illuminating the retina tissue 4. The images are acquired by two detectors (ie. sensor device 3). The predetermined illumination sequence comprises transpupillary pulses and transscleral pulses either in alternance or simultaneously as illustrated in figure 6 :
- one transpupillary pulse and one transscleral pulse simultaneously but with different wavelength, followed by
- 2 successive transscleral pulses;
This allows for instance the simultaneous acquisition of photoreceptors (with S1) and retinal pigment epithelium (with S2) on 2 different sensors by mean of source demultiplexing, or on the same detector by filtering the light sources at the pixel level. The sources S3 and S4 are for instance used to image the nerve fibre layer and the choriocapillaris on the same detector, thanks to the wavelength focus shift.

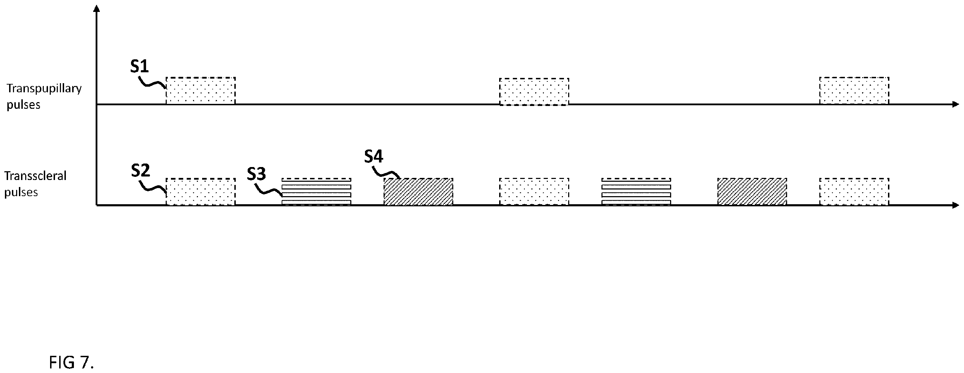

Figure 7 illustrates a third embodiment of a predetermined illumination sequence where illumination is provided by an illumination device 2 comprising a transpupillary illumination source S1 and three transscleral illumination sources S2, S3 and S4 illuminating the retina tissue 4. The images are acquired by one single detector (i.e. sensor device 3). The predetermined illumination sequence controlled by the controlled module 13 comprises transpupillary pulses and transscleral pulses either in alternance or simultaneously as illustrated in figure 6:
- one transpupillary pulse and one transscleral pulse simultaneously at the same wavelength, followed by
- 2 successive transscleral pulses;
This enables for instance the imaging of retinal vessels (with S1+S2) on a first focusing plane, the imaging of retinal nerve fibre layer (with S3) on the same focusing plane and retinal pigment epithelium (with S4) on a second focusing plane, the three type of structure on the same retina zone and on three distinct images made with a single detector.

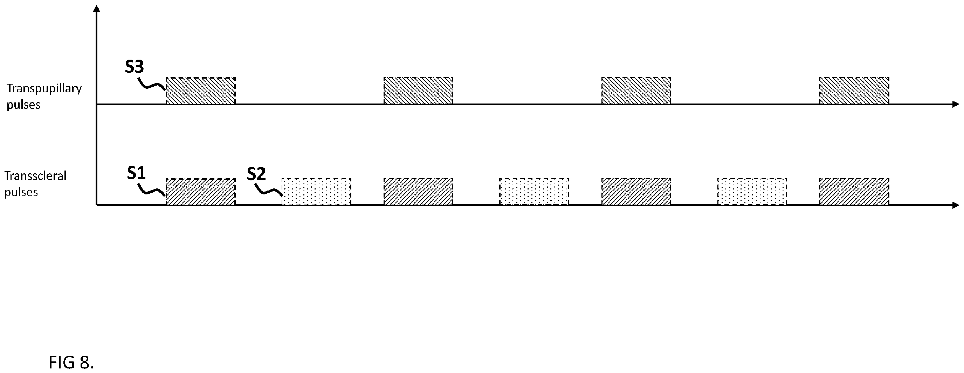

Figure 8 illustrates a third embodiment of a predetermined illumination sequence where illumination is provided by an illumination device 2 comprising a transpupillary illumination source S3 and two transscleral illumination source S1 and S2 illuminating the retina tissue 4. The images are acquired by two detectors (i.e. sensor device 3). The predetermined illumination sequence comprises transpupillary pulses and transscleral pulses either in alternance or simultaneously as illustrated in figure 6:
- one transpupillary pulse and one transscleral pulse simultaneously at the same wavelength, followed by
- 1 transscleral pulse;
This enables for instance the imaging of retinal vessels (with S1+S3) on a first detector over a small and detailed field of view and the imaging of retinal vessels (with S2) on a second detector over a larger field of view. It takes the advantage that transscleral illumination illuminate the retina over a large field of view by default, while it is difficult to illuminate a large field of view with transpupillary illumination because of the complex optical system needed to reach high numerical aperture in the illumination optics.

While the embodiments have been described in conjunction with a number of embodiments, it is evident that many alternatives, modifications and variations would be or are apparent to those of ordinary skill in the applicable arts. Accordingly, this disclosure is intended to embrace all such alternatives, modifications, equivalents and variations that are within the scope of this disclosure. This for example particularly the case regarding the different apparatuses which can be used.

### REFERENCE NUMBERS

- 1: System according to a first embodiment
- 2: Illumination device
- 3: Sensor device
- 4: Retina tissue
- 5: Eye
- 6: Illumination sources
- 7: Transscleral illumination source
- 8: Transpalpebral illumination source
- 9: Transpupillary illumination source
- 10: pupil
- 11: sclera
- 12: Surrounding ski
- 13: Control module

## Claims

1. Method for imaging the retina tissue (4) of a patient, the method comprising:
- i) providing a set of illumination sources (6) comprising at least a first illumination source (6,7) configured for emitting oblique illumination through the scattering tissue of the eye toward the retina tissue (4), for instance transscleral or transpalpebral illumination, and at least a second illumination source (9) configured for emitting transpupillary illumination toward the retina tissue (4);
- ii) defining a predetermined illumination sequence for controlling the oblique illumination and the transpupillary illumination of the retinal tissue (4) by selectively activating at least said first illumination sources (6,7) and said second illumination source (9) from said set of illumination sources (6);
- iii) illuminating the retinal tissue (4) according to the predetermined illumination sequence;
- iv) providing a sensor device (3) and synchronising the sensor device (3) according to the predetermined illumination sequence to ensure the acquisition of at least one image of the retina during illumination by said activated illumination sources (6).

2. Method according to claim 1, wherein the method further comprises:
- setting a focusing plane of the sensor device (3) during image acquisition at a specific depth in the retinal tissue (4), the focal plane being chosen depending on the type of structure of the retinal tissue (4) to be imaged;
- defining a predetermined illumination sequence for each focusing plane;

3. Method according to the preceding claim, wherein the structure being chosen in the list comprising the retinal pigment epithelium cells, the photoreceptors, the retinal nerve fibre layer, the retinal vessels, the retinal capillaries, the ganglion cells, the microglia, the amacrine cells, the lamina cribrosa, the choriocapillaris, the choroidal vessels, the choroid, the optic disc and the inner retina.

4. Method according to any one of claims 1 to 3, the method further comprising :
- Acquiring at least two images per activated illumination source (6);
- Processing the acquired images to provide at least one finale image provided by the first illumination source (7,8) and at least one finale image provided by the second illumination source (9).

5. Method according to any one of claims 1 to 4, wherein the predetermined illumination sequence comprises an alternate activation of at least two illumination sources from the set of illumination sources, preferably of at least the first illumination source (7,8) and the second illumination source (9).

6. Method according to any one of claims 1 to 5, wherein the predetermined illumination sequence comprises a simultaneous activation of at least two illumination sources (6) from the set of illumination sources, preferably of at least the first illumination source (7,8) and the second illumination source (9).

7. Method according to any one of claims 1 to 6, wherein at least two illumination sources (6) from the set of illumination sources have at least one identical optical parameter chosen among wavelength, spectrum, power on the retina.

8. Method according to any one of claims 1 to 7, wherein at least two illumination sources (6) from the set of illumination sources have different wavelength, or have the same wavelength.

9. Method according to any one of claims 1 to 8, wherein at least two illumination sources from the set of illumination sources emit in the infrared spectrum, preferably between about 700 nm and about 1000 nm.

10. Method according to the preceding claim, wherein the images are acquired at a constant focal plane.

11. System (1) for imaging the retina tissue (4) of a patient, the system (1) comprising an illumination device (2) and a sensor device (3), the illumination device (2) being configured for illuminating the retina tissue (4) while the sensor device (3) acquires images of the illuminated retina tissue (4),
the system (1) being **characterized in that** the illumination device (2) comprises
a set of illumination sources comprising at least a first illumination source (7,8) configured for emitting oblique illumination through the scattering tissue of the eye toward the retina tissue, for instance transscleral or transpalpebral illumination, and at least a second illumination source (9) configured for emitting transpupillary illumination toward the retina tissue (4),
the system (1) further comprising a control module (13) configured for controlling the oblique illumination and the transpupillary illumination of the retinal tissue (4) by selectively activating at least said first illumination sources (7,8) and said second illumination source (9) from said set of illumination sources according to a predetermined illumination sequence,
said control module (13) being further configured for synchronising the sensor device (3) according to the predetermined illumination sequence to ensure the acquisition of at least one image of the retina during illumination by said activated illumination sources.

12. System (1) according to claim 11, wherein the system (1) further comprises a correction module for correcting the optical aberrations of the eye of the patient, for instance chosen among a wavefront sensor, a wavefront corrector.

13. System (1) according to any one of claims 11 to 12, wherein the system (1) further comprises a processing module for processing the acquired images from the sensor device (3), the processing module being configured for processing images acquired from each illumination source to provide at least one finale image for each illumination source (6).

14. System (1) according to any one of claims 11 to 13, wherein the system (1) comprises a single detector (3) and the illumination sequence comprises an alternate activation of at least two illumination sources (6) from the set of illumination sources, or
the system (1) comprises one detector (3) per illumination source (6) and the illumination sequence comprises an alternate activation of at least two illumination sources (6) from the set of illumination sources (6).
